# EUROPÄISCHE PATENTSCHRIFT

(11) **EP 1 257 551 B1**
(45) Veröffentlichungstag und Bekanntmachung des Hinweises auf die Patenterteilung: **05.09.2007**
(21) Anmeldenummer: 01911528.6
(22) Anmeldetag: 25.01.2001
(51) Int. Cl.: C07D 471/04, A61K 31/437, A61P 25/00

(54) **HETEROZYKLISCHE VERBINDUNGEN UND DEREN ANWENDUNG ALS PARP-INHIBITOREN**
HETEROCYCLIC COMPOUNDS AND THEIR USE AS PARP INHIBITORS
COMPOSES HETEROCYCLIQUES ET LEUR UTILISATION COMME INHIBITEURS DE PARP

(30) Priorität: 01.02.2000 DE 10004238
(43) Veröffentlichungstag der Anmeldung: 20.11.2002
(73) Patentinhaber: Abbott GmbH & Co. KG, 65205 Wiesbaden (DE)
(72) Erfinder: LUBISCH, Wilfried, 69115 Heidelberg (DE); KOCK, Michael, 67105 Schifferstadt (DE); HÖGER, Thomas, 68535 Edingen-Neckarhausen (DE); GRANDEL, Roland, 69221 Dossenheim (DE); MÜLLER, Reinhold, 67105 Schifferstadt (DE); SCHULT, Sabine, 67346 Speyer (DE)
(74) Vertreter: Goldbach, Klara
(86) Internationale Anmeldenummer: PCT/EP2001/000790
(87) Internationale Veröffentlichungsnummer: WO 2001/057038

(56) Entgegenhaltungen:
- EP-A- 0 154 190
- WO-A-97/04771

## Beschreibung

Die vorliegende Erfindung betrifft neuartige heterozyklische Verbindungen, ihre Herstellung und die Verwendung als Inhibitoren des Enzyms Poly(ADP-ribose)polymerase oder PARP (EC 2.4.2.30) zur Herstellung von Arzneimitteln.

Poly (ADP-ribose)polymerase (PARP) bzw. wie es auch genannt wird Poly(ADP-ribose) synthase (PARS) stellt ein regulatorisches Enzym dar, das in Zellkernen gefunden wird (K. Ikai et al., J. Histochem. Cytochem. 1983, 31, 1261-1264). Man nimmt an, daß PARP eine Rolle bei der Reparatur von DNA-Brüchen spielt (M.S. Satoh et al., Nature 1992, 356, 356-358). Schädigungen oder Brüche der DNA-Stränge aktivieren das Enzym PARP, das, wenn es aktiviert ist, die Übertragung von ADP-Ribose aus NAD katalysiert (S. Shaw, Adv. Radiat. Biol., 1984, 11, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei einer Reihe von pathophysiologischen Zuständen beobachtet und man geht davon aus, daß diese Anhäufung von Radikalen zu den beobachteten Zell- bzw Organschäden führen oder...beitragen. Dazu zählen zum Beispiel ischämische Zustände von Organen wie im Schlaganfall, Herzinfarkt (C. Thiemermann et al., Proc. Natl. Acad. Sci. USA, 1997, 94, 679-683) oder Ischämie der Nieren, aber auch Reperfusionsschaden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von einer Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. Cancer Chemo. Pharmacol. 1988 22, 303). Nicht limitierende Beispiele für Tumore sind Leukämie, Glioblastome, Lymphome, Melanome, Mamma- und Zervikalkarzinome.

Zudem wurde gefunden, daß PARP-Inhibitoren immunosuppressive Wirkung zeigen können (D. Weltin et al. Int. J. Immunopharmacol. 1995, 17, 265-271).

Es wurde ebenfalls entdeckt, daß PARP bei immunologischen Erkrankungen bzw. Krankeiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. Inflammation 1996, 20, 203-215; W. Ehrlich et al. Rheumatol. Int. 1995, 15, 171-172; C.Szabo et al., Proc. Natl. Acad. Sci. USA 1998, 95, 3867-3872; S. Cuzzocrea et al. Eur. J. Pharmacol. 1998, 342, 67-76).

Weiterhin zeigte der PARP-Inhibitor 3-Aminobenzamid protektive Effekte in einem Modell für den Kreislaufschock (S. Cuzzocrea et al., Br. J. Pharmacol. 1997, 121, 1065-1074).

Ebenfalls gibt es experimentelle Hinweise, dass Inhibitoren des Enzymes PARP als Mittel zur Behandlung von Diabetes mellitus nützlich sein könnten (V. Burkart et al. Nature Med. 1999, 5, 314-319).

Unter PARP im Sinne der Erfindung, werden auch Isoenzyme des oben beschriebenen PARP-Enzyms verstanden. Solche Isoenzyme sind z.B. PARP II und PARP III, wie in WO99/64572 beschrieben.

Imidazopyridine bzw. Derivate dieser bizyklischen Verbindung stellen eine chemische Klasse dar, die vielfach in der organischen Synthese Verwendung fand. Ebenso sind bereits Derivate beschrieben worden, in denen ein Imidazopyridin am 6-Ring eine Carbonsäureamid-Grüppe trägt. In DE 2025427, DE 2611665, J. Reisch et al., J. Heterocycl. Chem. 1990, 27, 287-189 und Y. et al., J. Med. Chem. 1998, 41, 564-578, wurden Imidazopyridine hergestellt, die in 4-Stellung Carbonsäureamid-Gruppen tragen, die aber weiter am Carbonsäureamid-StickstoffAtom substituiert sind, und in 2-Stellung entweder nicht oder nur mit einfachen Resten-wie Methyl substituiert sind.

Imidazopyridine, die in 7-Stellung eine Carbonsäureamid-Gruppe tragen, sind wenig bekannt. Lediglich in Teulade et al. Eur. J. Med. Chem. Chim. Therap. 1979, 13, 271 ist das 1-Nitro-imidazo[1,2-a]pyridin-7-carbonsäureamid hergestellt worden.

Imidazopyridine, die in 6- und in 5-Stellung eine Carbonsäureamid-Gruppe tragen sind bereits mehrfach beschrieben worden. So sind hier auch Derivate bekannt, die in 2-Stellung aromatische Ringe tragen. Diese Verbindungen und weitere Derivate sind in L. Fisher et al. J. Med. Chem. 1972, 15, 982, Buu-Hoi et al. Bull. Soc. Chim Fr. 1961, 1344, und O.K. Kim et al. Bioorg. Med. Chem. Lett. 1997, 7, 27, beschrieben worden. In Buu-Hoi (siehe oben) und in BE 620141 wurden 2-Phenyl-imidazo[1,2-a]pyridine beschrieben, die in 6- bzw. 7-Stellung eine Carbonsäureamid-Gruppe tragen. In keinen der obigen Literaturzitate ist jedoch ein Hinweis zu finden, daß derartige Verbindungen eine therapeutische Verwendungsmöglichkeit haben können, die dadurch gegeben ist, daß diese Verbindungen Enzyme wie PARP hemmen.

Es wurde weiterhin überraschenderweise gefunden, daß heterozyklische Verbindungen wie zum Beispiel Imidazopyridin-Derivate gut wirksame Inhibitoren für das Enzym PARP darstellen. Des weiteren wurde gefunden, dass die erfindungsgemäßen Imidazo-[1,2-a]pyridin-Derivate, die in 4- bzw. 8-Stellung eine primäre Carbonsäureamid-Gruppe tragen, im Vergleich zu den Stellungsisomeren, die in 6- bzw. 7-Stellung eine Carbonsäureamid-Gruppe tragen, zum Beispiel in Buu-Hoi (siehe oben) und in BE 620141, gute Wirksamkeit in der Hemmung des Enzyms PARP zeigen, wogegen die Stellungsisomere nur sehr schlecht oder überhaupt nicht wirksam sind.

In der WO-A-97/04771 werden Benzimidazole beschrieben, denen eine Wirkung als PARP-Inhibitoren zugeschrieben wird.

EP-A-0 154 190 beschreibt Pyridone und deren Verwendung in der Behandlung von Krankheiten, insbesondere von Herzinsuffizienz und/oder Bluthochdruck.

In der vorliegenden Erfindung werden neue heterozyklische Verbindungen der allgemeinen Formel I beschrieben, die potente PARP-Inhibitoren darstellen.

Gegenstand der vorliegenden Erfindung sind heterozyklische Verbindungen der allgemeinen Formell 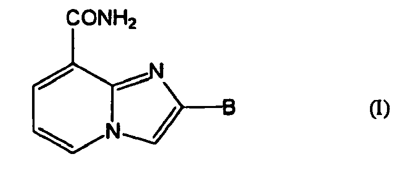 worin
- B: einen ungesättigten, gesättigten oder partial-ungesättigten mono-, bi- oder tri-cyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeuten kann, die jeweils noch mit einem R⁴ und maximal 3 gleichen oder verschiedenen Resten R⁵ substituiert sein können, und
- R⁴: Wasserstoff und -(D)ₚ-(E)ₛ-(F¹)_{q}-G¹-(F²)ᵣ-(G²)-G³ bedeutet,
wobei
- D: S, NR⁴³ und O
- E: Phenyl, , -SO₂- 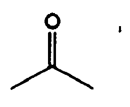 -S0₂NH-, -NHCO-, -CONH, -NHSO₂- und,
- s: 0 und 1 und
- G¹: eine Bindung bedeutet oder einen ungesättigten, gesättigten oder partialungesättigten mono-, bi- oder tricyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefel-atomen bedeuten kann, die jeweils noch mit maximal 3 unterschiedlichen oder gleichen Resten R⁵ substituiert sind, und ein oder zwei Kohlenstoff- bzw. Schwefel-Atome auch ein oder zwei =O-Gruppen tragen können, und
- G²: NR⁴¹R⁴² und 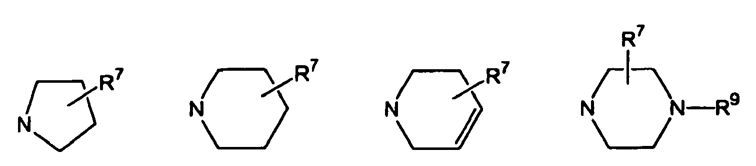 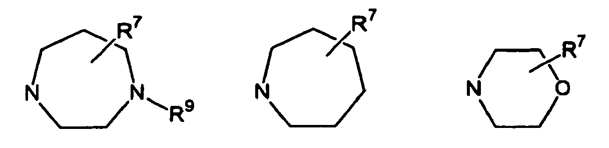 oder eine Bindung bedeutet und
- G³: einen ungesättigten, gesättigten oder partial-ungesättigten mono-, bi- oder tri-cyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeuten kann, die jeweils noch mit maximal 3 unterschiedlichen oder gleichen Resten R⁵ substituiert sind, und ein oder zwei Kohlenstoff- bzw. Schwefel-Atome auch ein oder zwei =O-Gruppen tragen können, oder Wasserstoff bedeutet, und
- p: 0 und 1 bedeuten kann und
- F¹: eine C₁-C₈-Alkylkette sein kann und
- F²: unabhängig von F¹ die gleiche Bedeutung wie F¹ besitzt
- q: 0 und 1 sein kann und
- r: 0 und 1 sein kann und
- R⁴¹: Wasserstoff. C₁-C₅-Alkyl, C₁-C₆-Alkyl-Phenyl, Phenyl, wobei jedes Kohlenstoffatom der Alkylketten und die Phenyl-Ringe noch maximal zwei Reste R⁶ tragen können, und (CH₂)ₜ-K und
- R⁴²: Wasserstoff, C₁-C₆-Alkyl, -CO₂-R⁸, -CO-R⁸. -SO₂-R⁸, -(C=NH)-R⁸ und -(C=NH)-NHR⁸ und
- R⁴³: Wasserstoff und C₁-C₄-Alkyl und
- t: 1, 2, 3, 4 und
- K: NR¹¹R¹², NR¹¹-C₁-C₄-Alkyl-Phenyl, Pyrrolidin, Piperidin, 1,2,5,6-Tetrahydropyridin, Morpholin, Homopiperidin, Homopiperazin, Piperazin, die noch mit einem C₁-C₆-Alkyl-Rest oder noch mit einem C₁-C₉-Alkyl-Phenyl-Rest und der Phenylrest noch mit maximal zwei R⁸¹ substituiert sein können, und
- R⁵: Wasserstoff, Chlor, Fluor, Brom, Jod, C₁-C₆-Alkyl, Nitro, CF₃, CN, NR¹¹R¹², NH-CO-R¹³, O-C₁-C₄-Alkyl
- R⁶: Wasserstoff, Chlor, Fluor, Brom, Jod, C₁-C₆-Alkyl, OH, Nitro, CF₃, CN, NR¹¹R¹², NH-CO-R¹³, O-C₁-C₄-Alkyl
- R⁷: Wasserstoff, C₁-C₆-Alkyl, Phenyl, wobei der Ring noch mit bis zu zwei gleichen oder verschiedenen Resten R⁷¹ substituiert sein kann, und ein Amin NR¹¹R¹² oder ein zyklisches gesättigtes Amin mit 3 bis 7 Gliedern, das noch mit einem Alkyl-Rest C₁-C₆-Alkyl substituiert sein kann, wobei die Reste R¹¹ und R¹² in K, R⁵, R⁶ und R⁷ unabhängig voneinander Wasserstoff oder C₁-C₄-Alkyl bedeuten und R¹³ in K, R⁵, R⁶ und R⁷ Wasserstoff, C₁-C₄-Alkyl, C₁-C₄-Alkyl-Phenyl oder Phenyl bedeutet,
- R⁷¹: OH, C₁-C₆-Alkyl, O-C₁-C₄-Alkyl, Chlor, Brom, Jod, Fluor, CF₃, Nitro, NH₂, und
- R⁸: C₁-C₆,-Alkyl, CF₃, Phenyl, C₁-C₄-Alkyl-Phenyl, wobei der Ring noch mit bis zu zwei Resten R⁸¹ substituiert sein kann, und
- R⁸¹: OH, C₁-C₆-Alkyl, O-C₁-C₄-Alkyl, Chlor, Brom, Jod, Fluor, CF₃, Nitro, NH₂, und
- R⁹: Wasserstoff, C₁-C₆-Alkyl, C₁-C₄-Alkyl-Phenyl, CO₂-C₁-C₄-Alkyl-Phenyl, CO₂-C₁-C₄-Alkyl, -SO₂-Phenyl, -COR⁸ und Phenyl, wobei die Phenyl-Ringe noch mit bis zu zwei gleichen oder verschiedenen Resten R⁹¹ substituiert sein können, und
- R⁹¹: OH, C₁-C₆-Alkyl, O-C₁-C₄-Alkyl, Chlor, Brom, Jod, Fluor, CF₃, Nitro und NH₂ sein kann,
sowie ihre tautomeren Formen, möglichen enantiomeren und diastereomeren Formen, und deren Phosphate, Carbamate von Aminosäuren und Ester.

Die Reste z.B. R⁵, R¹¹, R¹² und R¹³ können die verschiedenen Bedeutungen unabhängig voneinander und unabhängig von der jeweiligen Bedeutung bei einem anderen Rest (z.B. A¹, R⁵ etc.) annehmen.

Unter Cₓ-C_{y}-Alkyl wird immer soweit möglich verzweigtes und unverzweigtes Cₓ-C_{y}-Alkyl verstanden. Unverzweigtes Alkyl ist bevorzugt.

Bevorzugt werden die Verbindungen der Formel I, wobei
- X¹: ein N-Atom darstellt und
- X²: CH darstellt und
- R¹: Wasserstoff ist und
- A¹: CONH₂ darstellt und
- A²: Wasserstoff darstellt und
- B: Phenyl, Pyridin oder Piperidin darstellt, die jeweils noch mit einem Rest R⁴ und R⁵ substituiert sein können und
alle anderen Variablen die obige Bedeutung haben.
Die Verbindungen der Formel I können als Racemate, als enantiomerenreine Verbindungen oder als Diastereomere eingesetzt werden. Werden enantiomerenreine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd. 10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die in vivo in Verbindungen der allgemeinen Formel I metabolisiert werden. Typische Prodrugs sind Phosphate, Carbamate von Aminosäuren, Ester und andere.

Die Herstellung der erfindungsgemäßen, heterozyklischen Verbindungen 1 kann auf verschiedenen Wegen erfolgen.

Die möglichen Synthesemethoden sind im wesentlichen bereits bekannt oder orientieren sich an bekannten analogen Wegen.
Eine beispielhafte Methode ist im Syntheseschema 1 aufgeführt.

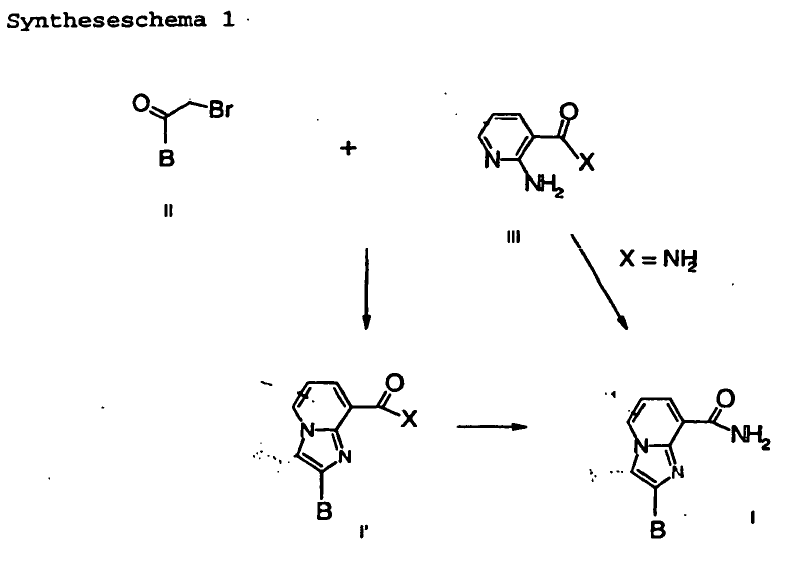

Ein α-Bromketon II und ein 2-Aminopyridin-Derivat III werden zu einem Imidazopyridin I' umgesetzt. Dabei arbeitet man bevorzugt in polaren Lösungsmitteln wie Aceton oder Alkoholen, gegebenenfalls unter Zusatz von Säuren wie Bromwasserstoffsäure, und zuerst bei Raumtemperatur und später bei erhöhter Temperatur, zum Beispiel bis zum Siedepunkt des eingesetzten Lösungsmittels. Alternativ kann man auch direkt bei erhöhter Temperatur arbeiten. Ist in III X eine NH₂-Gruppe, so erhält man auf dem obigen Wege die erfindungsgemäßen Verbindungen I. Ist dagegen X eine Estergruppe wie OCH₂CH₃, so wird I' (X = OCH₂CH₃) noch in I überführt. Das kann auf zwei Wegen erreicht werden.

I' (X = OCH₂CH₃) wird in Gemischen aus polaren Lösungsmitteln wie Tetrahydrofuran und Wasser oder in Wasser direkt unter Zusatz von Säuren wie Salzsäure oder Basen wie Natronlauge oder Lithiumhydroxid bei Raumtemperatur oder bei erhöhten Temperaturen, maximal der Siedetemperatur des Lösungsmittels, zu einer Carbonsäure I' (X = COOH) hydrolysiert. Diese Carbonsäure I' (X = COOH) kann anschließend mit ammoniakalischen Lösungen, zum Beispiel NH₃ in Alkoholen oder Wasser, in organischen Lösungsmitteln, bevorzugt in polaren, aprotischen Lösungsmitteln wie Tetrahydrofuran und Dimethylformamid, mittels gebräuchlichen Peptid-Kupplungsmethoden zum Carbonsäureamid umgesetzt werden, die wie im Schema 1 gezeigt die erfindungsgemäßen Verbindungen I darstellen. Einige Peptid-Kupplungsmethoden sind zum Beispiel im Houben-Weyl, Methoden der Organischen Chemie, 4. Aufl., E5, Kap. V bzw. C.R. Larock, Comprehensive Organic Transformations, VCH Publisher, 1989, Seite 972f. aufgelistet.

Ansonsten kann man, falls in I' X = 0-Alkyl ist, diesen Ester mit Ammoniak, bei gegebenenfalls erhöhter Temperatur und erhöhtem Druck, direkt zum erfindungsgemäßen Amid I umsetzten. Alternativ kann man den Ester I' (X = O-Alkyl) mit Hydrazin in polaren Lösungsmitteln wie den Alkoholen Butanol und Ethanol oder auch Dimethylformamid, bei erhöhten Temperaturen, vorzugsweise 80 bis 130°C, umsetzten, wobei ein Hydrazid I ('X = NHNH₂) anfällt, das danach noch unter reduktiven Bedingungen, wie mit Raney-Nickel in Alkoholen unter Rückfluß, zum erfindungsgemäßen Amid I reduziert werden kann.

Die in der vorliegenden Erfindung enthaltenen substituierten, heterozyklischen Verbindungen I stellen Inhibitoren des Enzyms Poly(ADP-ribose)polymerase oder PARP (EC 2.4.2.30) dar.

Die inhibitorische Wirkung der substituierten, heterozyklischen Verbindungen I wurde mit einem in der Literatur bereits bekannten Enzymtest ermittelt, wobei als Wirkmaßstab ein Ki-Wert ermittelt wurde. Die heterozyklischen Verbindungen I wurden in dieser Weise auf Hemmwirkung des Enzyms Poly(ADP-ribose)polymerase oder PARP (EC 2.4.2.30) gemessen.

Die substituierten heterozyklischen Verbindungen der allgemeinen Formeln I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten und/oder zu reduzierender Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die vorliegenden heterozyklischen Verbindungen der allgemeinen Formel I können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, verursacht durch medikamentöse Therapien wie z.B. bei der Cyclosporin-Behandlung, des akuten Nierenversagens oder von Schädigungen, die während und nach einer Nierentransplantation auftreten, dienen. Weiterhin können die Verbindungen der allgemeinen Formel I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten während und nach Herzklappenersatz, Aneurysmenresektionen und Herztransplantationen dienen. Ebenfalls können die vorliegenden Imidazopyridin-Derivate I zur Behandlung einer Revascularisation kritisch verengter Koronaraterien, zum Beispiel bei der PCTA und Bypass-Operationen, und kritisch verengter peripherer Arterien, zum Beispiel Beinarterien, dienen. Zudem können die heterozyklische Verbindungen I bei der Chemotherapie von Tumoren und deren Metastasierung nützlich sein und zur Behandlung von Entzündungen und rheumatischen Erkrankungen, wie z.B. rheumatischer Arthritis und auch zur Behandlung von Diabetes mellitus und zur Behandlung des Multiorganversagens, zum Beispiel beim septischen Schock, und zur Behandlung des ARDS ("acute respiratory distress-syndrom", Schocklunge) dienen.

Die erfindungsgemäßen Arzneimittelzubereitungen enthalten neben den üblichen Arzneimittelhilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon. Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel A: Hemmung des Enzyms Poly(ADP-ribose)polymerase oder PARP (EC 2.4.2.30)

Eine 96well Mikrotiterplatte (Falcon) wird mit Histonen (Type II-AS; SIGMA H7755) beschichtet. Histone werden dazu in Carbonat-Puffer (0,05 M NaHCO₃; pH 9.4) zu einer Konzentration von 50 µg/ml gelöst. Die einzelnen Wells der Mikrotiterplatte werden über Nacht mit je 100 µl dieser Histon Lösung inkubiert. Anschließend wird die Histon Lösung entfernt und die einzelnen Wells mit 200 µl einer 1%igen BSA (Bovine Serum Albumine) Lösung in Carbonat-Puffer für 2 Stunden bei Raumtemperatur inkubiert. Anschließend wird dreimal mit Waschpuffer (0,05 % Tween10 in PBS) gewaschen. Für die Enzymreaktion werden je Well 50 µl der Enzymreaktionslösung (5 µl Reaktions-Puffer (1M Tris-HCl pH 8,0, 100 mM MgCl₂, 10 mM DTT), 0,5 µl PARP (c = 0,22 µg/µl). 4 µl aktivierte DNA (SIGMA D-4522, 1 mg/ml in Wasser), 40,5 µl H₂O) mit 10 µl einer Inhibitorlösung für 10 Minuten vorinkubiert. Die Enzymreaktion wird durch Zugabe von 40 µl einer Substratlösung (4 µl Reaktion-Puffer (s.o.), 8 µl NAD-Lösung (100 µM in H₂O), 28 µl H₂O) gestartet. Reaktionszeit ist 20 Minuten bei Raumtemperatur. Die Reaktion wird durch dreimaliges Waschen mit Waschpuffer (s.o.) gestoppt. Anschließend folgt eine einstündige Inkubation bei Raumtemperatur mit einem spezifischen Anti-Poly-ADP-Ribose Antikörper inkubiert. Als Antikörper wurden ein monoklonaler anti-Poly-(ADP-ribose) Antikörpern "10H" (Kawamaitsu H et al. (1984) Monoclonal antibodies to poly (adenosine diphosphate ribose) recognize different structures. Biochemistry 23, 3771-3777) verwendet. Polyklonale Antikörper können ebenso verwendet werden.

Die Antikörper wurden in einer 1:5000 Verdünnung in AntikörperPuffer (1 % BSA in PBS; 0,05 % Tween20) eingesetzt. Nach dreimaligem Waschen mit Waschpuffer folgt eine einstündige Inkubation bei Raumtemperatur mit dem sekundärem Antikörper. Hier wurden für den monoklonalen Antikörper ein anti-Maus-IgG gekoppelt mit Peroxidase (Boehringer Mannheim) und für den Kaninchen Antikörper ein anti-Rabbit-IgG gekoppelt mit Peroxidase (SIGMA A-6154) jeweils in einer 1:10000 Verdünnung in Antikörperpuffer verwendet. Nach dreimaligem Waschen mit Waschpuffer erfolgt die Farbreaktion unter Verwendung von 100 µl/Well Farbreagenz (SIGMA, TMB-Fertigmix, T8540) für ca. 15 min. bei Raumtemperatur. Die Farbreaktion wird durch Zugabe von 100 µl 2M H₂SO₄ gestoppt. Danach wird sofort gemessen (450 nm gegen 620 nm; ELISA Platten Lesegerät "Easy Reader" EAR340AT, SLT-Labinstruments, Österreich). Der IC₅₀-Wert eines zu messenden Inhibitors liegt bei der Inhibitorkonzentration, wo eine halbmaximale Farbkonzentrationsänderung auftritt.

### Test von PARP-Inhibitoren in einem zellulären Assay

Zum Test der Wirkung von PARP-Inhibitoren werden eukaryontische Zellinien mit Chemikalien so behandelt, daß die DNA der Zellinie geschädigt und dadurch das in den Zellen vorhandene PARP-Enzym aktiviert wird. Durch die Aktivierung des Enzym werden Ketten von poly-ADP-Ribose (PAR) auf Proteinen gebildet. Diese Ketten werden von einem spezifischen Antikörper gebunden. Dieser wird wiederum von einem zweiten Antikörper gebunden, der mit einer fluoreszenten Markierung versehen ist. Die Fluoreszenz wird mit einem Fluoreszenzscanner gemessen und verhält sich zur Aktivität des Enzyms PARP proportional.PARP-Inhibitoren lassen sich an einer Abschwächung des Fluoreszenzsignals erkennen. Um Verfälschungen der Ergebnisse durch unterschiedliche Zellzahlen zu verhindern, wird die DNA der Zellen mit einem weiteren Farbstoff markiert und dessen Fluoreszenz ebenfalls im Fluoreszenzscanner bestimmt.

400000 Zellen der humanen Zellinie C4I werden in Zellkulturplatten mit 24 Kavitäten in RPMI-Medium mit 10 % fötalen Rinderserum bei 37°C, 5 % CO₂ bis zum Erreichen eines dichten Zellrasens bebrütet. Die Zellen werden mit DMEM gewaschen und die zu testenden PARP-Inhibitoren in verschiedenen Konzentrationen in DMEM zugegeben. Nach einer Inkubation für 20 min bei 37°C wird mit Wasserstoffperoxid eine Konzentration von 1mM eingestellt und weitere 10min bei 37°C inkubiert. Zur Kontrolle werden Zellen in einigen Kavitäten nicht mit Wasserstoffperoxid behandelt (keine PARP-Aktivierung) oder erhalten keinen Inhibitor (maximale PARP-Aktivierung). Die Zellen werden einmal mit PBS gewaschen und durch Zugabe von auf -20°C vorgekühltem Methanol/Aceton Gemisch (7 Teile Methanol, 3 Teile Aceton) 10 min bei -20°C fixiert. Danach werden die Zellen getrocknet, durch Zugabe von PBS für 10 min bei Zimmertemperatur rehydratisiert und unspezifische Bindungsstellen in PBS mit 0,05 % Tween20 und 5 % Trockenmilchpulver für 30 min bei Zimmertemperatur blockiert. Der Maus anti-PAR Antikörper wird in einer Konzentration von 20 µg/ml in PBS mit 0,05 % Tween20 und 5 % Trockenmilchpulver zugegeben und 1 h bei 37°C inkubiert. Nicht gebundener Antikörper wird durch fünfmaliges Waschen mit PBS für jeweils 5 min entfernt. Anschließend wird mit einem verdünnten Ziege anti-Maus FITC-gekoppelten Zweitantikörper (Verdünnung 1:50 in PBS mit 0,05 % Tween20, 5 % Trockenmilchpulver und 1 ag/m1 DAPI (4',6-Diamidino-2-Phenyl-indol)) für 30 min bei 37°C inkubiert. Nicht gebundener Antikörper wird durch fünfmaliges Waschen mit PBS für jeweils 5 min entfernt. Die FITC- und DAPI-Fluoreszenzen werden an mehreren Stellen der Kavitäten mit Hilfe eines Fluoreszenzscanners gemessen. Zur Auswertung wird das FITC-Signal auf das DAPI-Signal normiert. Die Berechnung der IC50-Werte erfolgt nach halblogarithmischer Auftragung der normierten Werte der verschiedenen Inhibitorkonzentrationen.

### Beispiele

### Beispiel 1:

### 2-Phenyl-imidazo[1,2-a]pyridin-8-carbonsäureamid

### a) 2-Phenyl-imidazo[1,2-a]pyridin-8-carbonsäureethylester x HBr

2,0 (12 mMol) 2-Aminonikotinsäureethylester und 2,4 g (12 mMol) 2'-Bromacetophenon wurden 100 ml Aceton gegeben und für 16 Stunden bei Raumtemperatur gerührt. Anschließend wurde alles für 3 stunden unter Rückfluß gekocht. Nach dem Abkühlen wurde alles im Vakuum eingeengt. Der Rückstand wurde in 100 ml Methanol gerührt und danach vorsichtig mit 1 ml 47%iger Bromwasserstoffsäure in Eisessig versetzt. Alles wurde für eine Stunde unter Rückfluß gekocht und anschließend wieder im Vakuum eingeengt. Dieser Rückstand wurde in möglichst wenig Methanol gelöst und mit 3 ml 47%iger Bromwasserstoffsäure versetzt. Dann wurde vorsichtig bis zur beginnenden Trübung mit Ether versetzt, wonach das Produkt langsam auskristallisierte. Man erhielt 2,5 g.

### b) 2-Phenyl-imidazo[1,2-a]pyridin-8-carbonsäure

1,9 g (5,5 mMol) des Zwischenproduktes 1a wurden in 50 ml Methanol gelöst. Nach der Zugabe von 70 ml 4M Natronlauge wurde alles für zwei Stunden unter Rückfluß gekocht. Nach dem Abkühlen wurde das organische Lösungsmittel im Vakuum entfernt. Der dabei anfallende Niederschlag wurde abgesaugt und in heißem Wasser gelöst. Diese Lösung wurde vorsichtig mit verdünnter Salzsäure neutralisiert und über Nacht gekühlt. Dabei fiel das Produkt aus. Man erhielt 0,71 g.

### c) 2-Phenyl-imidazo[1,2-a]pyridin-8-carbonsäureamid

0,7 g (2, 9 mMol) des Zwischenproduktes 1b und-0,4 g (2,9 mMol) 1-Hydroxy-1H-benzotriazol x Hydrat wurden in 40 ml wasserfreiem Dimethylformamid gelöst und mit 150 ml einer 0,5 M Ammoniak-Lösung in Dioxan versetzt. Anschließend wurden bei 10°C 0,56 g (2,9 mMol) N'-3-Dimethylaminopropyl)-N-ethyl-carbodiimid zugegeben und alles für 16 Stunden bei Raumtemperatur gerührt. Diese Reaktionslösung wurde im Vakuum eingeengt. Der Rückstand wurde danach zwischen wäßriger Natriumhydrogenkarbonat-Lösung und Essigester verteilt. Die organische Phase wurde abgetrennt, nochmals mit Wasser gewaschen, getrocknet und im Vakuum eingeengt. Der so erhaltene Rückstand wurde mit wenig Methylenchlorid behandelt, wobei das Produkte auskristallisierte. Man erhielt 0,25 g des Produktes.
1_{H-NMR} (D₆-DMSO): δ = 7.1 (H), 7.35(1H), 7.5(2H), 8.0(4H), 8.6(1H), 8.8(1H) und 9.7 (1H)ppm.

### Beispiel 2

### 2-(4-Nitrophenyl-imidazo[1,2-a]pyridin-8-carbonsäureamid

Das Produkt wurde analog dem Beispiel 1 aus 2-Aminonikotinsäureethylester und 2'- Brom-4-nitroacetophenon hergestellt.
¹H-NMR (D₆-DMSO): 8 = 7.1(1H), 8.1(1H), 8.2(1H), 8.3(4H), 8.8(2H) und 9.5(1H) ppm.

### Beispiel 3

### 2-(4-Aminophenyl)-imidazo[1,2-a]pyridin-8-carbonsäureamid

Das Produkt aus Beispiel 1 wurde in Alkohol gelöst und nach Zugabe von Palladium/Kohle mit Wasserstoff hydriert. Anschließend wurde filtriert und das Filtrat im Vakuum eingeengt. Man erhielt das Produkt.
¹H-NMR (D₆-DMSO): δ = 5.35(2H), 6.7(2H), 7.0(1H), 7.7(2H), 7.9(2H), 8.2(1H), 8.7(1H) und 9.8(1H) ppm.

### Beispiel 4

### 2-(2-Benzothienyl)-imidazo[1,2-a]pyridin-8-carbonsäureamid

Das Produkt wurde analog der Vorschrift aus Beispiel 1 unter Einsatz des Benzothiophen-2-carboxaldehyds hergestellt.
¹H-NMR (D₆-DMSO): δ= 7.1(1H), 7.5(2H), 8.1(3H), 8.4(1H), 8.5(1H), 8.7(1H), 8.8(1H) und 9.6(1H) ppm.

### Beispiel 5

### 2-(4-Bromphenyl)-imidazo[1,2-a]pyridin-8-carbonsäureamid

Das Produkt wurde analog dem Beispiel 1 aus 2-Aminonikotinsäureethylester und 2'-Brom-4-bromacetophenon hergestellt.
¹H-NMR **(D₆-DNSO):** δ = 7.1(1H), 7.7(2H), 7.9-8.1(3H), 8.1(1H, breit), 8.6(1H), 8.75(1H), 9.55(1H, breit).

### Beispiel 6

### 2-(4-Imidazol-1-yl-phenyl)-imidazo[1,2-a]pyridin-8-carbonsäureamid

Das Produkt wurde analog dem Beispiel 1 aus 2-Aminonikotinsäureetyhlester und 2'-Brom-4-(imidazol-1-yl)-acetophenon hergestellt.
¹H-NMR (D₆-DMSO): δ = 7.0-7.2(2H), 7.75-7.95(3H), 8.05(2H), 8.1(2H), 8.25(1H), 8.7(1H), 8.8(1H), 9.6(1H).

Folgende erfindungsgemäße Verbindungen können analog den oben beschriebenen Methoden hergestellt werden:
1. 2-(4(4-n-Propyl-piperazin-1-yl)-phenyl)-imidazo[1,2-a]-pyridin-8-carbonsäureamid
2. 2-(4-Piperazin-1-yl-phenyl)- imidazo[1,2-a]pyridin-8-carbonsäureamid
3. 2-(4(4-Isopropyl-piperazin-1-yl)-phenyl)-imidazo[1,2-a]-pyridin-8-carbonsäureamid
4. 2-(4(4-Henzyl-piperazin-1-yl)-phenyl)-imidazo[1,2-a]pyridin-8-carbonsäureamid
5. 2-(4(4-n-Butyl-piperazin-1-yl)-phenyl)-imidazo[1,2-a]pyridin-8-carbonsäureamid
6. 2-(4(4-Ethyl-piperazin-1-yl)-phenyl)-imidazo[1,2-a]pyridin-8-carbonsäureamid
7. 2-(4-(2-N,N-Dimethylamino-eth-1-yloxy)-phenyl)-imidazo-[1,2-a]pyridin-8-carbonsäureamid
8. 2-(4-(2-Pyrrolidinl-yl-eth-1-yloxy)-phenyl)-imidazo[1,2-a]-pyridin-8-carbonsäureamid
9. 2-(4-(2-Piperidin-1-yl-eth-1-yloxy)-phenyl)-imidazo[1,2-a]-pyridin-8-carbonsäureamid
10. 2-(4-(2-Piperazin-1-yl-eth-1-yloxy)-phenyl)-imidazo[1,2-a]-pyridin-8-carbonsäureamid
11. 2-(4-(2-(4-Methyl-piperazin-1-yl)-eth-1-yloxy)-phenyl)-imidazo[1,2-a]pyridin-8-carbonsäureamid
12. 2-(4-(2-(4-Propyl-piperazin-1-yl)-eth-1-yloxy)-phenyl-imidazo[1,2-a]pyridin-8-carbonsäureamid
13. 2-(4- (2-(4-Ethyl-piperazin-1-yl)-eth-1-yloxy)-phenyl-imidazo[1,2-a]pyridin-8-carbonsäureamid
14. 2-(4-(2-(4-Benzyl-piperazin-1-yl)-eth-1-yloxy)-phenyl-imidazo[1,2-a]pyridin-8-carbonsäureamid
15. 2-(4-(2-(4-Acetamido-piperazin-1-yl)-eth-1-yloxy)-phenyl-imidazo[1,2-a]pyridin-8-carbonsäureamid
16. 2-(4-(2-(4-Benzamido-piperazin-1-yl)-eth-1-yloxy)-phenyl-imidazo[1,2-a]pyridin-8-carbonsäureamid
17. 2-(4-Homopiperazin-1-yl-phenyl)-imidazo[1,2-a]pyridin-8-carbonsäureamid
18. 2-(4(4-Methylhomopiperazin-1-yl)-phenyl)-imidazo[1,2-a]-pyridin-8-carbonsäureamid
19. 2-(4(4-Benzylhomopiperazin-I-yl)-phenyl)-imidazo[1,2-a]-pyridin-8-carbonsäureamid
20. 2-(4-(4-n-Butyl-homopiperazin-1-yl)-phenyl)-imidazo[1,2-a]-pyridin-8-carbonsäureamid
21. 2-(4(4-Ethylhomo-piperazin-1-yl)-phenyl)-imidazo[1,2-a]-pyridin-8-carbonsäureamid
22. 2-(4-Methoxy-phenyl)-imidazo[1,2-a]pyridin-8-carbonsäureamid
23. 2-(4-Methyl-phenyl)-imidazo[1,2-a]pyridin-8-carbonsäureamid
24. 2-(4-Phenyl-phenyl)-imidazo[1,2-a]pyridin-8-carbonsäureamid
25. 2-(4-Isopropyl-phenyl)-imidazo[1,2-a]pyridin-8-carbonsäureamid
26. 2-(4-Flour-phenyl)-imidazo[1,2-a]pyridin-8-carbonsäureamid
27. 2-(4-Triflourmethyl-phenyl)- imidazo[1,2-a]pyridin-8-carbonsäureamid
28. 2-(3-Methoxy-phenyl)-imidazo[1,2-a]pyridin-8-carbonsäureamid
29. 2-(3-Chlor-phenyl)-imidazo[1,2-a]pyridin-8-carbonsäureamid
30. 2-(3-Amino-phenyl)-imidazo[1,2-a]pyridin-8-carbonsäureamid
31. 2-(3-Methyl-phenyl)-imidazo[1,2-a]pyridin-8-carbonsäureamid
32. 2-(3-Phenyl-phenyl)-imidazo[1,2-a]pyridin-8-carbonsäureamid
33. 2-(3-Isopropyl-phenyl)-imidazo[1,2-a]pyridin-8-carbonsäureamid
34. 2-(3-Flour-phenyl)-imidazo[1,2-a]pyridin-8-carbonsäureamid
35. 2-(3-Triflourmethyl-phenyl)-imidazo[1,2-a]pyridin-8-carbonsäureamid
36. 2-Piperidin-4-yl-imidazo[1,2-a]pyridin-8-carbonsäureamid
37. 2-(1-Methyl-piperidin-4-yl)-imidazo[1,2-a]pyridin-8-carbonsäureamid
38. 2-(1-Ethyl-piperidin-4-yl)-imidazo[1,2-a]pyridin-8-carbonsäureamid
39. 2-(1-n-Propyl-piperidin-4-yl)-imidazo[1,2-a]pyridin-8-carbonsäureamid
40. 2-(1-Benzyl-piperidin-4-yl)-imidazo[1,2-a]pyridin-8-carbonsäureamid
41. 2-(1-n-Butyl-piperidin-4-yl)-imidazo[1,2-a]pyridin-8-carbonsäureamid
42. 2-(1-Isopropyl-piperidin-4-yl)-imidazo[1,2-a]pyridin-8-carbonsäureamid
43. 2-Pyridin-4-yl-imidazo [1,2-a]pyridin-8-carbonsäureamid
44. 2-Pyridin-3-yl-imidazo[1,2-a]pyridin-8-carbonsäureamid
45. 2-Pyridin-2-yl-imidazo[1,2-a]pyridin-8-carbonsäureamid
46. 2-Thien-2-yl-imidazo[1,2-a]pyridin-8-carbonsäureamid
47. 2-Thien-3-yl-imidazo[1,2-a]pyridin-8-carbonsäureamid
48. 2-Indol-3-yl-imidazo[1,2-a]pyridin-8-carbonsäureamid
49. 2-Indol-5-yl-imidazo[1,2-a]pyridin-8-carbonsäureamid
50. 2-Indol-2-yl-imidazo[1,2-a]pyridin-8-carbonsäureamid
51. 2-Chinolin-3-yl-imidazo[1,2-a]pyridin-8-carbonsäureamid
52. 2-Chinolin-2-yl-imidazo[1,2-a]pyridin-8-carbonsäureamid
53. 2-Chinolin-4-yl-imidazo[1,2-a]pyridin-8-carbonsäureamid
54. 2-Isochinolin-1-yl-imidazo[1,2-a]pyridin-8-carbonsäureamid
55. 2-Isochinolin-3-yl-imidazo[1,2-a]pyridin-8-carbonsäureamid
56. 2-Chinoxalin-2-yl-imidazo[1,2-a]pyzidin-8-carbonsäureamid
57. 2-Naphth-2-yl-imidazo[1,2-a]pyridin-8-carbonsäureamid
58. 2-Naphth-1-yl-imidazo[1,2-a]pyridin-8-carbonsäureamid
59. 2-(4-(2(N,N-Dimethylamino)-eth-1-ylamino)-phenyl)-imidazo-[1,2-a]pyridin-8-carbonsäureamid
60. 2-(4-(2(N,N-Diethylamino)-eth-1-ylamino)-phenyl)-imidazo-[1,2-a]pyridin-8-carbonsäureamid
61. 2-(4-(2-Piperidin-1-yl-eth-1-ylamino)-phenyl)-imidazo[1,2-a]-pyridin-8-carbonsäureamid
62. 2-(4-(2-Pyrrolidin-1-yl-eth-1-ylamino)-phenyl)-imidazo[1,2-a]-pyridin-8-carbonsäureamid
63. 2- (4- (3 (N,N-Dimethylamino)- prop-1-ylamino)-phenyl)-imidazo-[1,2-a]pyridin-8-carbonsäureamid
64. 2-(4- (3 (N,N-Diethylamino) -prop-1-ylamino) -phenyl) -imidazo-[1,2-a]pyridin-8-carbonsäureamid
65. 2- (4- (3-Piperidin-1-yl-prop-1-ylamino) -phenyl) -imidazo [1,2-a]-pyridin-8-carbonsäureamid
66. 2- (4- (3-Pyrrolidin-1-yl-prop-1-ylamino)-phenyl)-imidazo-[1, 2-a]pyridin-8-carbonsäureamid
67. 2-Cyclohexyl-imidazo[1,2-a]pyridin-8-carbonsäureamid
68. 2- (cis-4-Amino-cyclohex-1-yl)-imidazo[1,2-a]pyridin-8-carbonsäureamid
69. 2-(4-Methoxy-cyclohex-1-yl)-imidazo[1,2-a]pyridin-8-carbonsäureamid
70. 2-(4-(2(N,N-Dimethylamino)-eth-1-yl-methylamino)-phenyl)-imidazo[1,2-a]-pyridin-8-carbonsäureamid
71. 2-(4-(4-Methyl-piperazin-1-yl)-phenyl)-imidazo[1,2-a]pyridin-8-carbonsäureamid

## Patentansprüche

1. Verbindungen der Formel I 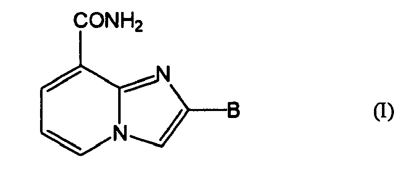 worin
B einen ungesättigten, gesättigten oder partial-ungesättigten mono-, bi-oder tricyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeuten kann, die jeweils noch mit einem R⁴ und maximal 3 gleichen oder verschiedenen Resten R⁵ substituiert sein können, und
R⁴ Wasserstoff und -(D)ₚ-(E)ₛ-(F¹)_{q}-G¹-(F²)ᵣ-(G²)-G³ bedeutet,
wobei
D S, NR⁴³ und O
E Phenyl, 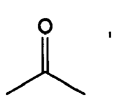 -SO₂-, -SO₂NH-, -NHCO-, -CONH-, -NHSO₂- und,
s 0 und 1 und
G¹ eine Bindung bedeutet oder einen ungesättigten, gesättigten oder partial-ungesättigten mono-, bi- oder tricyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeuten kann, die jeweils noch mit maximal 3 unterschiedlichen oder gleichen Resten R⁵ substituiert sind, und ein oder zwei Kohlenstoffbzw. Schwefel-Atome auch ein oder zwei =0-Gruppen tragen können, und
G² NR⁴¹R⁴² und 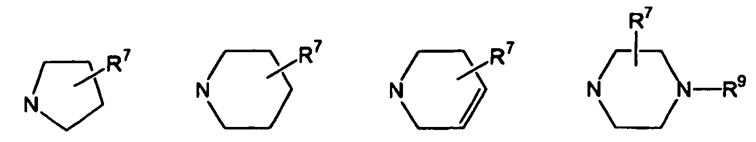 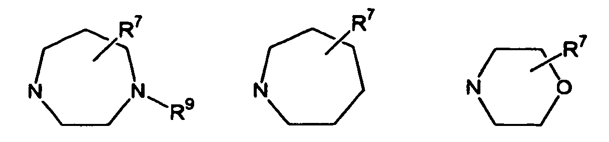 oder eine Bindung bedeutet und
G³ einen ungesättigten, gesättigten oder partial-ungesättigten mono-, bi-oder tricyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeuten kann, die jeweils noch mit maximal 3 unterschiedlichen oder gleichen Resten R⁵ substituiert sind, und ein oder zwei Kohlenstoff- bzw. Schwefel-Atome auch ein oder zwei =O-Gruppen tragen können, oder Wasserstoff bedeutet, und
p 0 und 1 bedeuten kann und
F¹ eine C₁-C₈-Alkylkette sein kann und
F² unabhängig von F¹ die gleiche Bedeutung wie F¹ besitzt
q 0 und 1 sein kann und
r 0 und 1 sein kann und
R⁴¹ Wasserstoff, C₁-C₆-Alkyl, C₁-C₆-Alkyl-Phenyl, Phenyl, wobei jedes Kohlenstoffatom der Alkylketten und die Phenyl-Ringe noch maximal zwei Reste R⁶ tragen können, und (CH₂)ₜ-K und
R⁴² Wasserstoff, C₁-C₆-Alkyl, -CO₂-R⁸, -CO-R⁸, -SO₂-R⁸, -(C=NH)-R⁸ und - (C=NH)-NHR⁸ und
R⁴³ Wasserstoff und C₁-C₄-Alkyl und
t 1,2,3,4und
K NR¹¹R¹², NR¹¹-C₁-C₄-Alkyl-Phenyl, Pyrrolidin, Piperidin, 1,2,5,6-Tetrahydropyridin, Morpholin, Homopiperidin, Homopiperazin, Piperazin, die noch mit einem C₁-C₆-Alkyl-Rest oder noch mit einem C₁-C₆-Alkyl-Phenyl-Rest und der Phenylrest noch mit maximal zwei R⁸¹ substituiert sein können, und
R⁵ Wasserstoff, Chlor, Fluor, Brom, Jod, C₁-C₆-Alkyl, Nitro, CF₃, CN, NR¹¹R¹², NH-CO-R¹³, O-C₁-C₄-Alkyl
R⁶ Wasserstoff, Chlor, Fluor, Brom, Jod, C₁-C₆-Alkyl, OH, Nitro, CF₃, CN, NR¹¹R¹², NH-CO-R¹³, O-C₁-C₄-Alkyl
R⁷ Wasserstoff, C₁-C₆-Alkyl, Phenyl, wobei der Ring noch mit bis zu zwei gleichen oder verschiedenen Resten R⁷¹ substituiert sein kann, und ein Amin NR¹¹R¹² oder ein zyklisches gesättigtes Amin mit 3 bis 7 Gliedern, das noch mit einem Alkyl-Rest C₁-C₆-Alkyl substituiert sein kann, wobei die Reste R¹¹ und R¹² in K, R⁵, R⁶ und R⁷ unabhängig voneinander Wasserstoff oder C₁-C₄-Alkyl bedeuten und R¹³ in K, R⁵, R⁶ und R⁷ Wasserstoff, C₁-C₄-Alkyl, C₁-C₄-Alkyl-Phenyl oder Phenyl bedeutet,
R⁷¹ OH, C₁-C₆-Alkyl, O-C₁-C₄-Alkyl, Chlor, Brom, Jod, Fluor, CF₃, Nitro, NH₂, und
R⁸ C₁-C₆-Alkyl, CF₃, Phenyl, C₁-C₄-Alkyl-Phenyl, wobei der Ring noch mit bis zu zwei Resten R⁸¹ substituiert sein kann, und
R⁸¹ OH, C₁-C₆-Alkyl, O-C₁-C₄-Alkyl, Chlor, Brom, Jod, Fluor, CF₃, Nitro, NH₂, und
R⁹ Wasserstoff, C₁-C₆-Alkyl, C₁-C₄-Alkyl-Phenyl, CO₂-C₁-C₄-Alkyl-Phenyl, CO₂-C₁-C₄-Alkyl, -SO₂-Phenyl, -COR⁸ und Phenyl, wobei die Phenyl-Ringe noch mit bis zu zwei gleichen oder verschiedenen Resten R⁹¹ substituiert sein können, und
R⁹¹ OH, C₁-C₆-Alkyl, O-C₁-C₄-Alkyl, Chlor, Brom, Jod, Fluor, CF₃, Nitro und NH₂ sein kann,
sowie ihre tautomeren Formen, möglichen enantiomeren und diastereomeren Formen, und deren Phosphate, Carbamate von Aminosäuren und Ester.

2. Verbindungen der Formel I nach Anspruch 1, wobei
B Phenyl, Pyridin oder Piperidin darstellt, die jeweils noch mit einem Rest R⁴ und R⁵ substituiert sein können.

3. Verbindungen der Formel I nach Anspruch 1 oder 2 wobei
R⁴ Wasserstoff oder -D_{0,1}-F¹_{0,1}-G²-G³ mit G³ gleich Wasserstoff und G² keine Bindung bedeutet und
D O und NR⁴³, wobei R⁴³ Wasserstoff und C₁-C₃-Alkyl und
F¹ C₂-C₄-Alkyl bedeutet.

4. Verwendung von Verbindungen der Formel I nach einem der Ansprüche 1 bis 3 zur Herstellung von Arzneimitteln zur Behandlung von neurodegenerativen Krankheiten und neuronalen Schädigungen.

5. Verwendung nach Anspruch 4 zur Behandlung von solchen neurodegenerativen Krankheiten und neuronalen Schädigungen, die durch Ischämie, Trauma oder Massenblutungen ausgelöst werden.

6. Verwendung nach Anspruch 4 zur Behandlung des Schlaganfalls und des Schädel-Himtraumas.

7. Verwendung nach Anspruch 4 zur Behandlung der Alzheimerschen Krankheit der Parkinsonsche Krankheit und der Huntington-Krankheit.

8. Verwendung von Verbindungen der Formel I nach einem der Ansprüche 1 und 3 zur Herstellung von Arzneimitteln zur Behandlung oder Prophylaxe von Schädigungen durch Ischämien.

9. Verwendung von Verbindungen der Formel I nach einem der Ansprüche 1 und 3 zur Herstellung von Arzneimitteln zur Behandlung von Epilepsien.

10. Verwendung von Verbindungen der Formel I nach Anspruch 9 zur Herstellung von Arzneimitteln zur Behandlung von generalisierten epileptischen Anfällen, partiell epileptischen Anfällen und komplex-partiellen Anfällen.

11. Verwendung von Verbindungen der Formel I nach einem der Ansprüche 1 und 3 zur Herstellung von Arzneimitteln zur Behandlung von Schädigungen der Nieren nach renalen Ischämien, Schädigungen, die durch medikamentöse Therapie verursacht werden und zur Behandlung während und nach Nierentransplantationen.

12. Verwendung von Verbindungen der Formel I nach Anspruch 11 zur Herstellung von Arzneimitteln zur Behandlung von Schädigungen, die durch die Cyclosporin-Therapie verursacht werden.

13. Verwendung von Verbindungen der Formel I nach einem der Ansprüche 1 und 3 zur Herstellung von Arzneimitteln zur Behandlung von Schädigungen des Herzens nach cardialen Ischämien.

14. Verwendung von Verbindungen der Formel I nach einem der Ansprüche 1 und 3 zur Herstellung von Arzneimitteln zur Behandlung von Mikroinfarkten.

15. Verwendung von Verbindungen der Formel I nach einem der Ansprüche 1 und 3 zur Herstellung von Arzneimitteln zur Behandlung bei einer Revascularisation kritisch verengter Koronararterien oder kritisch verengter peripherer Arterien, insbesondere Beinarterien.

16. Verwendung von Verbindungen der Formel I nach einem der Ansprüche 1 und 3 zur Herstellung von Arzneimitteln zur Behandlung des akuten Myocardinfarktes und von Schädigungen während und nach dessen medikamentöser oder mechanischer Lyse.

17. Verwendung von Verbindungen der Formel I nach einem der Ansprüche 1 und 3 zur Herstellung von Arzneimitteln zur Behandlung von Tumoren und deren Metastasierung.

18. Verwendung von Verbindungen der Formel I nach einem der Ansprüche 1 und 3 zur Herstellung von Arzneimitteln zur Behandlung von Sepsis, des Multiorganversagens.

19. Verwendung von Verbindungen der Formel I nach einem der Ansprüche 1 und 3 zur Herstellung von Arzneimitteln zur Behandlung von immunologischen Krankheiten wie Entzündungen und rheumatische Erkrankungen.

20. Verwendung von Verbindungen der Formel I nach einem der Ansprüche 1 und 3 zur Herstellung von Arzneimitteln zur Behandlung von Diabetes mellitus.

21. Arzneimittel enthaltend neben Trägerstoffen eine oder mehrere Verbindungen der Formel I nach Anspruch 1 bis 3.

22. Verwendung von Verbindungen der Formel I nach einem der Ansprüche 1 bis 3 zur Herstellung von Arzneimitteln zur Behandlung von Erkrankungen mit erhöhter oder zu reduzierender PARP-Aktivität.

## Claims

1. Compounds of the formula I 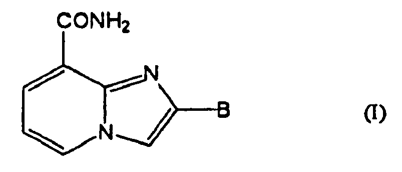 wherein
B can denote an unsaturated, saturated or partly unsaturated mono-, bi- or tricyclic ring having a maximum of 15 carbon atoms, an unsaturated, saturated or partly unsaturated mono-, bi- or tricyclic ring having a maximum of 14 carbon atoms and 0 to 5 nitrogen atoms, 0 to 2 oxygen atoms or 0 to 2 sulfur atoms, each of which can also be substituted by an R⁴ and a maximum of 3 identical or different radicals R⁵, and
R⁴ denotes hydrogen and - (D)ₚ- (E)ₛ-(F₁) _{q}-G¹- (F²)ᵣ- (G²) - _{G}³
wherein
D denotes S, NR⁴³ and O
E denotes phenyl, 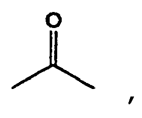 -SO₂-, -SO₂NH-, -NHCO-, -CONH-, -NHSO₂- and
s denotes 0 and 1 and
G¹ denotes a bond or can denote an unsaturated, saturated or partly unsaturated mono-, bi- or tricyclic ring having a maximum of 15 carbon atoms, an unsaturated, saturated or partly unsaturated mono-, bi- or tricyclic ring having a maximum of 14 carbon atoms and 0 to 5 nitrogen atoms, 0 to 2 oxygen atoms or 0 to 2 sulfur atoms, each of which can also be substituted by a maximum of 3 different or identical radicals R⁵, and one or two carbon or sulfur atoms can also carry one or two =0 groups, and
G² denotes NR⁴¹R⁴² and 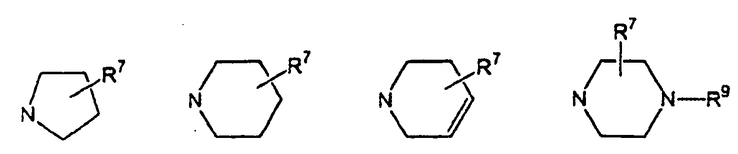 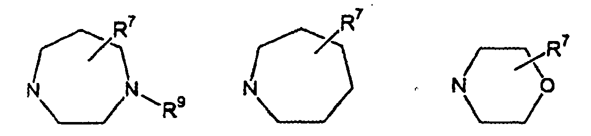 or a bond and
G³ can denote an unsaturated, saturated or partly unsaturated mono-, bi- or tricyclic ring having a maximum of 15 carbon atoms, an unsaturated, saturated or partly unsaturated mono-, bi- or tricyclic ring having a maximum of 14 carbon atoms and 0 to 5 nitrogen atoms, 0 to 2 oxygen atoms or 0 to 2 sulfur atoms, each of which can also be substituted by a maximum of 3 different or identical radicals R⁵, and one or two carbon or sulfur atoms can also carry one or two =0 groups, or denotes hydrogen and
p can denote 0 and 1 and
F¹ can be a C₁-C₈-alkyl chain and
F² independently of F¹ has the same meaning as F¹
q can be 0 and 1 and
r can be 0 and 1 and
R⁴¹ can be hydrogen, C₁-C₆-alkyl, C₁-C₆-alkyl-phenyl, phenyl, wherein each carbon atom of the alkyl chains and the phenyl rings can also carry a maximum of two radicals R⁶, and (CH₂)ₜ-K and
R⁴² can be hydrogen, C₁-C₆-alkyl, -CO₂-R⁸, -CO-R⁸, SO₂-R⁸, -(C-NH)-R⁸ and -(C=NH)-NHR⁸ and
R⁴³ can be hydrogen and C₁-C₄-alkyl and
t can be 1, 2, 3, 4 and
K can be NR¹¹R¹², NR¹¹- C₁-C₄-alkyl-phenyl, pyrrolidine, piperidine, 1,2,5,6-tetrahydropyridine, morpholine, homopiperidine, homopiperazine, piperazine, which can also be substituted by a C₁-C₆-alkyl radical or also by a C₁-C₆-alkyl-phenyl radical and the phenyl radical can also be substituted by a maximum of two R⁸¹, and
R⁵ can be hydrogen, chlorine, fluorine, bromine, iodine, C₁-C₆-alkyl, nitro, CF₃, CN, NR¹¹R¹², NH-CO-R¹³, O-C₁-C₄-alkyl
R⁶ can be hydrogen, chlorine, fluorine, bromine, iodine, C₁-C₆-alkyl, OH, nitro, CF₃, CN, NR¹¹R¹², NH-CO-R¹³, O-C₁-C₄-alkyl
R⁷ can be hydrogen, C₁-C₆-alkyl, phenyl, wherein the ring can also be substituted by up to two identical or different radicals R⁷¹, and an amine NR¹¹R¹² or a cyclic saturated amine having 3 to 7 members, which can also be substituted by an alkyl radical C₁-C₆-alkyl, wherein the radicals R¹¹ and R¹² in K, R⁵, R⁶ and R⁷ independently of one another denote hydrogen or C₁-C₄-alkyl and R¹³ in K, R⁵, R⁶ and R⁷ denotes hydrogen, C₁-C₄-alkyl, C₁-C₄-alkyl-phenyl or phenyl,
R⁷¹ can be OH, C₁-C₆-alkyl, O-C₁-C₄-alkyl, chlorine, bromine, iodine, fluorine, CF₃, nitro, NH₂ and
R⁸ can be C₁-C₆-alkyl, CF₃, phenyl, C₁-C₄-alkyl-phenyl, wherein the ring can also be substituted by up to two radicals R⁸¹, and
R⁸¹ can be OH, C₁-C₆-alkyl, O-C₁-C₄-alkyl, chlorine, bromine, iodine, fluorine, CF₃, nitro, NH₂ and
R⁹ can be hydrogen, C₁-C₆-alkyl, C₁-C₄-alkyl-phenyl, CO₂-C₁-C₄-alkyl-phenyl, CO₂-C₁-C₄-alkyl, -SO₂-phenyl, -COR⁸ and phenyl, wherein the phenyl rings can also be substituted by up to two identical or different radicals R⁹¹, and
R⁹¹ can be OH, C₁-C₆-alkyl, O-C₁-C₄-alkyl, chlorine, bromine, iodine, fluorine, CF₃, nitro and NH₂,
and their tautomeric forms, possible enantiomeric and diastereomeric forms, and phosphates thereof, carbamates thereof of amino acids and esters thereof.

2. Compounds of the formula 1 according to claim 1, wherein
B represents phenyl, pyridine or piperidine, each of which can also be substituted by a radical R⁴ and R⁵.

3. Compounds of the formula 1 according to claim 1 or 2, wherein
R⁴ denotes hydrogen or -D_{0,1}-F¹_{0,1}-G²-G³, where G³ is hydrogen and G² does not denote a bond and
D denotes 0 and NR⁴³, wherein R⁴³ denotes hydrogen and C₁-C₃-alkyl and
F¹ denotes C₂-C₄-alkyl.

4. Use of compounds of the formula I according to one of claims 1 to 3 for the preparation of medicaments for treatment of neurodegenerative diseases and neuronal damage.

5. Use according to claim 4, for treatment of those neurodegenerative diseases and that neuronal damage induced by ischaemia, trauma or massive haemorrhages.

6. Use according to claim 4, for treatment of stroke and cranio-cerebral trauma.

7. Use according to claim 4, for treatment of Alzheimer's disease, Parkinson's disease and Huntington's disease.

8. Use of compounds of the formula I according to one of claims 1 and 3 for the preparation of medicaments for treatment or prophylaxis of damage due to ischaemias.

9. Use of compounds of the formula I according to one of claims 1 and 3 for the preparation of medicaments for treatment of epilepsies.

10. Use of compounds of the formula I according to claim 9 for the preparation of medicaments for treatment of generalized epileptic fits, partly epileptic fits and complex partly epileptic fits.

11. Use of compounds of the formula I according to one of claims 1 and 3 for the preparation of medicaments for treatment of damage to the kidneys following renal ischaemias, damage cause by medicamentous therapy and for treatment during and after kidney transplants.

12. Use of compounds of the formula I according to claim 11 for the preparation of medicaments for treatment of damage caused by cyclosporin therapy.

13. Use of compounds of the formula I according to one of claims 1 and 3 for the preparation of medicaments for treatment of damage to the heart following cardiac ischaemias.

14. Use of compounds of the formula I according to one of claims 1 and 3 for the preparation of medicaments for treatment of microinfarctions.

15. Use of compounds of the formula I according to one of claims 1 and 3 for the preparation of medicaments for treatment in the event of revascularization of critically narrowed coronary arteries or critically narrowed peripheral arteries, in particular leg arteries.

16. Use of compounds of the formula I according to one of claims 1 and 3 for the preparation of medicaments for treatment of acute myocardial infarction and of damage during and after medicamentous or mechanical lysis thereof.

17. Use of compounds of the formula I according to one of claims 1 and 3 for the preparation of medicaments for treatment of tumours and metastasing thereof.

18. Use of compounds of the formula I according to one of claims 1 and 3 for the preparation of medicaments for treatment of sepsis, and of multiple organ failure.

19. Use of compounds of the formula I according to one of claims 1 and 3 for the preparation of medicaments for treatment of immunological diseases, such as inflammations and rheumatic diseases.

20. Use of compounds of the formula 1 according to one of claims 1 and 3 for the preparation of medicaments for treatment of diabetes mellitus.

21. Medicaments comprising, in addition to carrier substances, one or more compounds of the formula according to claim 1 to 3.

22. Use of compounds of the formula I according to one of claims 1 to 3 for the preparation of medicaments for treatment of diseases with increased PARP activity or PARP activity which is to be reduced.

## Revendications

1. Composés de formule 1 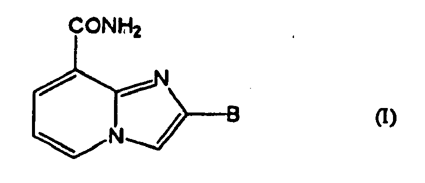 dans laquelle
B peut représenter un système cyclique mono-, bi- ou tricyclique insaturé, saturé ou partiellement insaturé ayant au plus 15 atomes de carbone, un système cyclique mono-, bi- ou tricyclique saturé, insaturé ou partiellement insaturé ayant au plus 14 atomes de carbone et 0 à 5 atomes d'azote, 0 à 2 atomes d'oxygène ou 0 à 2 atomes de soufre, qui peuvent encore être substitués chacun par un R⁴ et au plus 3 restes R⁵ identiques ou différents, et
R⁴ représente l'hydrogène et -(D)ₚ-(E)ₛ-(F¹)_{q}-G¹-(F²)ᵣ-(G²)-G³,
où
D est S, NR⁴³ et O,
E représente phényle, 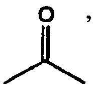 -SO₂-, -SO₂NH-, -NHCO-, -CONH-, -NHSO₂- et
s est 0 ou 1 et
G¹ représente une liaison ou peut représenter un système cyclique mono-, bi- ou tricyclique insaturé, saturé ou partiellement insaturé ayant au plus 15 atomes de carbone, un système cyclique mono-, bi- ou tricyclique saturé, insaturé ou partiellement insaturé ayant au plus 14 atomes de carbone et 0 à 5 atomes d'azote, 0 à 2 atomes d'oxygène ou 0 à 2 atomes de soufre, qui peuvent encore être substitués chacun par au plus 3 restes R⁵ identiques ou différents, et un ou deux atomes de carbone ou de soufre peuvent aussi porter un ou deux groupes =O, et
G² représente NR⁴¹R⁴² et 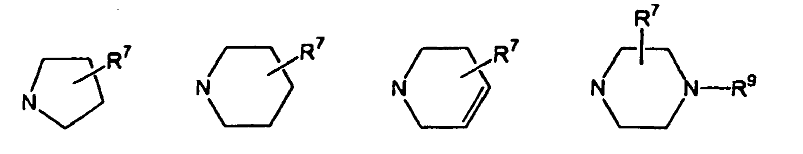 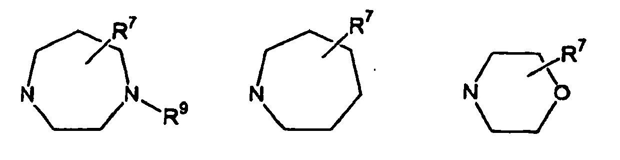 ou une liaison, et
G³ peut représenter un système cyclique mono-, bi- ou tricyclique insaturé, saturé ou partiellement insaturé ayant au plus 15 atomes de carbone, un système cyclique mono-, bi- ou tricyclique saturé, insaturé ou partiellement insaturé ayant au plus 14 atomes de carbone et 0 à 5 atomes d'azote, 0 à 2 atomes d'oxygène ou 0 à 2 atomes de soufre, qui peuvent encore être substitués chacun par au plus 3 restes R⁵ identiques ou différents, et un ou deux atomes de carbone ou de soufre peuvent aussi porter un ou deux groupes =0, ou l'hydrogène, et
p peut représenter 0 et 1, et
F¹ peut être une chaîne alkyle en C₁-C₈, et
F² a, indépendamment de F¹, la même signification que F¹,
q peut être 0 et 1, et
r peut être 0 et 1, et
R⁴¹ peut être hydrogène, alkyle en C₁-C₆, (alkyl en C₁-C₆)-phényle, phényle, chaque atome de carbone des chaînes alkyle et les cycles phényle pouvant encore porter au plus deux restes R⁶, et (CH₂)ₜ-K, et
R⁴² peut être hydrogène, alkyle en C₁-C₆, -CO₂-R⁸, -CO-R⁸, -SO₂-R⁸, -(C=NH)-R⁸ et -(C=NH)-NHR⁸, et
R⁴³ peut être hydrogène et alkyle en C₁-C₄, et
t peut être 1, 2, 3, 4 et
K peut être NR¹¹R¹², NR¹¹-(alkyl en C₁-C₄)-phényle, pyrrolidine, pipéridine, 1,2,5,6-tétrahydropyridine, morpholine, homopipéridine, homopipérazine, pipérazine, qui peuvent encore être substitués par un reste alkyle en C₁-C₆ ou encore par un reste (alkyl en C₁-C₆)-phényle, et le reste phényle peut encore être substitué par au plus deux R⁸¹, et
R⁵ peut être hydrogène, chloro, fluoro, bromo, iodo, alkyle en C₁-C₆, nitro, CF₃, CN, NR¹¹R¹² NH-CO-R¹³ O-alkyle en C₁-C₄,
R⁶ peut être hydrogène, chloro, fluoro, bromo, iodo, alkyle en C₁-C₆, OH, nitro, CF₃, CN, NR¹¹R¹², NH-CO-R¹³, O-alkyle en C₁-C₄,
R⁷ peut être hydrogène, alkyle en C₁-C₆, phényle, le cycle pouvant encore être substitué par jusqu'à 2 restes R⁷¹ identiques ou différents, et une amine NR¹¹ R¹² ou une amine cyclique saturée de 3 à 7 chaînons qui peut encore être substituée par un reste alkyle en C₁-C₆, les restes R¹¹ et R¹² dans K, R⁵, R⁶ et R⁷ signifiant indépendamment les uns des autres hydrogène ou alkyle en C₁-C₄, et R¹³ dans K, R⁵, R⁶ et R⁷ signifiant hydrogène, alkyle en C₁-C₄, (alkyle en C₁-C₄)-phényle ou phényle,
R⁷¹ peut être OH, alkyle en C₁-C₆, O-alkyle en C₁-C₄, chloro, bromo, iodo, fluoro, CF₃, nitro, NH₂, et
R⁸ peut être alkyle en C₁-C₆, CF₃, phényle, (alkyl en C₁-C₄)-phényle, le cycle pouvant encore être substitué par jusqu'à 2 restes R⁸¹, et
R⁸¹ peut être OH, alkyle en C₁-C₆, O-alkyle en C₁-C₄, chloro, bromo, iodo, fluoro, CF₃, nitro, NH₂, et
R⁹ peut être hydrogène, alkyle en C₁-C₆, (alkyl en C₁-C₄)-phényle, CO₂-(alkyl en C₁-C₄)-phényle, CO₂-(alkyle en C₁-C₄), -SO₂-phényle, -COR⁸ et phényle, les cycles phényle pouvant encore être substitués par jusqu'à 2 restes R⁹¹ identiques ou différents, et
R⁹¹ peut être OH, alkyle en C₁-C₆, O-alkyle en C₁-C₄, chloro, bromo, iodo, fluoro, CF₃, nitro et NH₂,
ainsi que leurs formes tautomères, les formes énantiomères et diastéréoisomères possibles, et leurs phosphates, carbamates d'aminoacides et esters.

2. Composés de formule I selon la revendication 1, dans lesquels
B représente phényle, pyridine ou pipéridine, qui peuvent chacun être encore substitués par un reste R⁴ et R⁵.

3. Composés de formule I selon la revendication 1 ou 2, dans lesquels
R⁴ représente hydrogène ou D_{0,1}-F¹_{0,1}-G²-G³, où G³ est l'hydrogène et G² ne représente pas de liaison, et
D représente O et NR⁴³, où R⁴³ est hydrogène et alkyle en C₁-C₃, et
F¹ représente alkyle en C₂-C₄.

4. Utilisation de composés de formule I selon l'une des revendications 1 à 3 pour la préparation de médicaments destinés au traitement de maladies neurodégénératives et de lésions neuronales.

5. Utilisation selon la revendication 4 pour le traitement de maladies neurodégénératives et de lésions neuronales qui sont déclenchées par une ischémie, un traumatisme ou une hémorragie massive.

6. Utilisation selon la revendication 4 pour le traitement de l'attaque cérébrale et du traumatisme crâniocérébral.

7. Utilisation selon la revendication 4 pour le traitement de la maladie d'Alzheimer, de la maladie de Parkinson et de la maladie de Huntington.

8. Utilisation de composés de formule I selon l'une des revendications 1 et 3 pour la préparation de médicaments destinés au traitement ou à la prophylaxie de lésions dues à des ischémies.

9. Utilisation de composés de formule I selon l'une des revendications 1 et 3 pour la préparation de médicaments destinés au traitement d'épilepsies.

10. Utilisation de composés de formule 1 selon la revendication 9 pour la préparation de médicaments destinés au traitement de crises épileptiques généralisées, de crises épileptiques partielles et de crises complexes partielles.

11. Utilisation de composés de formule I selon l'une des revendications 1 et 3 pour la préparation de médicaments destinés au traitement de lésions des reins après des ischémies rénales, de lésions qui sont entraînées par une thérapie médicamenteuse, et au traitement pendant et après des transplantations rénales.

12. Utilisation de composés de formule I selon la revendication 11 pour la préparation de médicaments destinés au traitement de lésions entraînées par une thérapie avec une cyclosporine.

13. Utilisation de composés de formule I selon l'une des revendications 1 et 3 pour la préparation de médicaments destinés au traitement de lésions du coeur après des ischémies cardiaques.

14. Utilisation de composés de formule 1 selon l'une des revendications 1 et 3 pour la préparation de médicaments destinés au traitement de microinfarctus.

15. Utilisation de composés de formule 1 selon l'une des revendications 1 et 3 pour la préparation de médicaments destinés au traitement lors d'une revascularisation d'artères coronaires rétrécies de manière critique ou d'artères périphériques rétrécies de manière critique, en particulier d'artères des jambes.

16. Utilisation de composés de formule I selon l'une des revendications 1 et 3 pour la préparation de médicaments destinés au traitement de l'infarctus aigu du myocarde et de lésions pendant et après sa lyse médicamenteuse ou mécanique.

17. Utilisation de composés de formule I selon l'une des revendications 1 et 3 pour la préparation de médicaments destinés au traitement de tumeurs et de leurs métastases.

18. Utilisation de composés de formule I selon l'une des revendications 1 et 3 pour la préparation de médicaments destinés au traitement de la septicémie et du choc septique.

19. Utilisation de composés de formule I selon l'une des revendications 1 et 3 pour la préparation de médicaments destinés au traitement de maladies immunologiques comme des inflammations et des maladies rhumatismales.

20. Utilisation de composés de formule I selon l'une des revendications 1. et 3 pour la préparation de médicaments destinés au traitement du diabète sucré.

21. Médicaments contenant, outre des supports, un ou plusieurs composés de formule I selon les revendications 1 à 3.

22. Utilisation de composés de formule I selon l'une des revendications 1 à 3 pour la préparation de médicaments destinés au traitement de maladies avec une activité de PARP élevée ou à réduire.
